# EUROPEAN PATENT APPLICATION

(11) **EP 2 982 391 A1**
(43) Date of publication of application: **10.02.2016**
(21) Application number: 14779392.1
(22) Date of filing: 20.03.2014
(51) Int. Cl.: A61L 31/00, A61F 2/82

(54) **STENT**

(30) Priority: 05.04.2013 JP 2013079325
(71) Applicant: Terumo Kabushiki Kaisha, Tokyo 151-0072 (JP)
(72) Inventor: BABA, Takeshi, Fujinomiya-shi Shizuoka 418-0015 (JP)
(74) Representative: Casalonga
(86) International application number: PCT/JP2014/057907
(87) International publication number: WO 2014/162904

(57) **Abstract**

According to the present invention, there is provided a stent which can suppress galvanic corrosion while radiopacity is sufficiently ensured. The stent according to the present invention includes a sintered body of a base material that is formed of a first metal material with a second metal material that is dispersed in the base material. The second metal material has a specific gravity higher than that of the first metal material, and has a corrosion potential expressed by a corrosion potential of the first metal material ± 200 mV.

## Description

### Technical Field

The present invention relates to a stent, and particularly to a living body indwelling stent. Specifically, the present invention relates to a stent which suppresses galvanic corrosion while sufficiently ensuring radiopacity, and particularly to a living body indwelling stent.

### Background Art

Conventionally, since a treatment method is less invasive to a patient, angina pectoris, myocardinal infarction, or the like has been treated, for example, by using percutaneous transluminal coronary angioplasty (PTCA) for a stenosis in coronary arteries, and ischemic diseases or the like in femoral arteries or carotid arteries have been treated, for example, by using percutaneous transluminal angioplasty (PTA) for a stenosis in these arteries. Both of these treatment methods need procedures of ensuring and resuming blood flow by using a catheter having a small folded balloon mounted on a distal end and by expanding a stenosed or occluded blood vessel. In order to prevent the expanded blood vessel from being restenosed or occluded, treatment has been performed in which patency of the blood vessel is ensured by causing a metal stent to indwell the blood vessel. The stent is generally cut out from a single metal pipe, or is formed of a metal wire in a mesh shape, a coil shape, and the like. In any case, the stent has a tubular structure which can be contracted. The stent in a contracted state is inserted into the blood vessel by using a catheter, and is caused to indwell the stenosis after being expanded so as to mechanically support a lumen in the blood vessel. In addition, in some cases, a radiopaque metal material is installed in an end portion of the stent as a marker so that a position of the stent can be satisfactorily confirmed under X-ray fluoroscopy.

Incidentally, each metal has an inherent potential. If dissimilar metals are brought into contact with each other and are immersed in an electrolyte solution, since the potentials of both metals are different from each other, a potential difference (galvanic potential) is generated between a less-noble material (metal having a strong ionization tendency) and a noble metal (metal having a weak ionization tendency). Consequently, a battery (local battery, galvanic battery) is formed, and a current (local current) flows therein, thereby causing corrosion. The corrosion occurring in response to an electrochemical reaction due to the formation of the local battery in which these dissimilar metals serve as electrodes is called galvanic corrosion, dissimilar metal contact corrosion or local current corrosion.

As a metal material configuring the stent, stainless steel (for example, SS316L), a cobalt-based alloy, a nickel-titanium alloy, or the like is generally used. In addition, as the radiopaque marker, gold or platinum which is excellent in radiopacity is generally used. However, gold or platinum has a very high potential. For this reason, a potential difference increases between the metal materials used for the stent and gold or platinum used for the radiopaque marker. Consequently, the above-described galvanic corrosion occurs, and the less noble metal is eluted. Therefore, when the stent indwells a living body for a long period of time, there is a possibility that the metal may be eluted in a body fluid such as the blood and the like. In view of the above-described problem, PTL 1 discloses a method in which a radiopaque tab is installed in an end portion of the stent by micro-alloying the radiopaque metal material such as gold, platinum, and the like with the nickel-titanium alloy configuring the stent (Paragraph [0038]). PTL 1 discloses that this tab does not significantly generate a galvanic element (Paragraph [0016]).

In addition, PTL 2 discloses a radiopaque stent including a cylindrical body portion formed of a cobalt-chromium alloy containing multiple radiopaque materials. PTL 2 discloses that a highly visible image can be obtained by alloying these radiopaque materials with the cobalt-chromium alloy (Paragraph [0017]).

### Citation List

### Patent Literature

PTL 1: JP-A-2002-263195 (corresponding to U.S. Patent Application Publication No. 2001/0001317)
PTL 2: JP-T-2003-527931 (corresponding to International Publication No. 01/72349)

### Summary of Invention

### Technical Problem

However, according to PTL 1 described above, the amount of the radiopaque metal material which is added to the metal material (nickel-titanium alloy) configuring the stent is negligible. Therefore, sufficient radiopacity cannot be provided for the stent. Accordingly, when the stent is caused to indwell, a position of the stent is not satisfactorily visible under X-ray fluoroscopy. On the other hand, if the amount of the radiopaque metal material which is added to the metal material configuring the stent is increased in order to improve the radiopacity, micro-alloying is no longer expected, and phase separation or precipitation occurs in many cases. Eventually, the potential difference (galvanic potential) is generated between the metal material configuring the stent and the radiopaque metal material, thereby accelerating the galvanic corrosion. In addition, in a case of the stent in which the radiopaque marker is disposed only in the end portion as described above, it is not possible to recognize an overall shape of the stent. Consequently, there is also a problem in that difficulties are encountered when treatment is performed by using an overlapping method in which multiple stents are caused to indwell while respective end portions overlap each other in a longitudinal direction, or by using a two-stent method or the like in which the stent is caused to indwell both a main vessel and a branch vessel of the blood vessel in a state where the stents are brought into contact with each other.

In the stent disclosed in PTL 2 described above, the radiopaque material is fully alloyed with a base material of the stent. Therefore, since the radiopaque material is entirely uniformly present, the overall image of the stent is not sufficiently clearly captured, and is indistinct under X-ray fruoroscopy. Therefore, in order to improve visibility under X-ray fluoroscopy, it is necessary to increase the amount of the radiopaque material. However, the increased amount is undesirable in terms of miniaturization of the stent since a thickness or a width of the stent increases. In addition, phase separation or precipitation occurs depending on a type of metal, or the base material of the stent is corroded, thereby causing a possibility that the strength thereof may become poor. For this reason, according to the stent disclosed in PTL 2 described above, there are limitations on a type or an added amount of the radiopaque material which can be used. Accordingly, it is difficult to design the stent.

Therefore, the present invention is made in view of the above-described circumstances, and an object thereof is to provide a stent which can suppress galvanic corrosion while radiopacity is sufficiently ensured.

### Solution to Problem

In order to solve the above-described problem, through a result from intensive studies, the present inventor has found that the above-described object can be achieved by bringing a metal having a potential close to a metal material configuring a stent body into a dispersed state and by installing the metal material in the stent body. In this manner, the present invention is completely made.

That is, the above-described problem is solved by providing a stent including a sintered body of a base material that is formed of a first metal material with a second metal material that is dispersed in the base material. The second metal material has a specific gravity higher than that of the first metal material, and has a corrosion potential expressed by a corrosion potential of the first metal material ± 200 mV.

### Brief Description of Drawings

[Fig. 1] Fig. 1 is a view illustrating a stent according to an embodiment of the present invention. In Fig. 1, the reference numeral 1 represents the stent, and the reference numeral 2 represents a stent body, respectively.
[Fig. 2] Fig. 2 is a partial enlarged view of a portion 3 surrounded by a dotted line in the stent illustrated in Fig. 1. In Fig. 2, the reference numeral 4 represents a second metal material.
[Fig. 3] Fig. 3 is a graph illustrating a galvanic current (nA/cm²) of 75Mo-25Ta and Au in Reference Example 2.
[Fig. 4] Fig. 4 is a graph illustrating a galvanic current (nA/cm²) of 50Mo-50Ta and Au in Reference Example 2.
[Fig. 5] Fig. 5 is a graph illustrating a galvanic current (nA/cm²) of 50Ta-50W and Au in Reference Example 2.
[Fig. 6] Fig. 6 is a graph illustrating a galvanic current (nA/cm²) of 25Nb-75W and Au in Reference Example 2.
[Fig. 7] Fig. 7 is a view illustrating a device for measuring a galvanic current in Reference Example 2. In Fig. 7, the reference numeral S1 represents a counter electrode, the reference numeral S2 represents a sample electrode, the reference numeral S3 represents a reference electrode, the reference numeral D represents a cell, the reference numeral I represents a zero shunt ammeter, and the reference numeral E represents an electrometer, respectively.

### Description of Embodiments

Hereinafter, an embodiment of the present invention will be described. The present invention is not limited only to the following embodiment. In some cases, dimensional proportions in the drawings may be exaggerated for convenience of description and different from actual proportions. In addition, in this description, "X to Y" indicating a range means "equal to or greater than X and equal to or less than Y", a "weight" and a "mass", "wt%" and "mass%", and "pts.wt." and "pts.mass" are treated as a synonym. In addition, unless otherwise specified, operations, physical properties, and the like are measured under conditions of room temperature (20°C to 25°C) / relative humidity of 40% to 50%.

According to the present invention, there is provided a stent including a sintered body of a base material that is formed of a first metal material with a second metal material that is dispersed in the base material. The second metal material has a specific gravity higher than that of the first metal material, and has a corrosion potential expressed by a corrosion potential of the first metal material ± 200 mV. The stent having the above-described configuration can suppress galvanic corrosion while radiopacity is sufficiently ensured. Therefore, the stent according to the present invention is satisfactorily visible under X-ray fluoroscopy, and can be caused to safely indwell a living body lumen such as blood vessels or the like for a long period of time.

As described above, if dissimilar metals are brought into contact with each other and are immersed in an electrolyte solution, since potentials of both metals are different from each other, a potential difference (galvanic potential) is generated between a less-noble material (metal having a strong ionization tendency) and a noble metal (metal having a weak ionization tendency). Consequently, a battery (local battery, galvanic battery) is formed, and a current (local current) flows therein, thereby causing corrosion (galvanic corrosion). Then, as the potential difference becomes greater, the current flowing therein further increases, thereby accelerating the corrosion. On the other hand, if the potentials included in the metal materials (first metal material and second metal material) configuring the stent are substantially the same as each other, galvanic corrosion does not occur in principle. A method of alloying is conceivable in order to adjust the potential difference. However, in many cases, merely alloying causes phase separation, precipitation, or the like as described above. Consequently, it is difficult to adjust the potential.

In contrast, the stent according to the present invention adopts a configuration in which the second metal material having a corrosion potential within a specific range with respect to the first metal material (metal) configuring the base material (stent body) and having a specific gravity higher than that of the first metal material (metal) is dispersed in the base material (stent body).

Here, since the second metal material has the specific gravity higher than that of the first metal material (metal) (is excellent in radiopacity), the second metal material acts as a radiopaque marker of the stent. In addition, the second metal material has a potential (corrosion potential) which is close to that of the first metal material (for example, stainless steel, a Co-based alloy, or the like) configuring the base material (stent body). Therefore, according to the stent of the present invention, the potential difference is small between the base material (first metal material) and the radiopaque marker (second metal material). Accordingly, even if the radiopaque marker comes into contact with the base material (first metal material configuring the stent body) which is a dissimilar metal, the local current substantially hardly flows therein. Therefore, it is possible to suppress or prevent metal elution caused by the galvanic corrosion.

In addition, in a dispersed state, the second metal material is present in the base material (stent body). Therefore, according to the stent of the present invention, compared to the stent which is alloyed as disclosed in PTL 2, the radiopaque marker itself is present while being dispersed in the stent body. Accordingly, it is possible to further improve visibility under X-ray fluoroscopy, and it is possible to accurately recognize an overall shape of the stent. For example, in some cases, treatment is performed by using an overlapping method in which multiple stents are caused to indwell while respective end portions overlap each other in a longitudinal direction, or by using a two-stent method in which the stent is caused to indwell both a main vessel and a branch vessel of the blood vessel in a state where the stents are brought into contact with each other, or the like. However, in these cases, it is necessary to accurately recognize a position of the stent. However, according to the conventional stent, as described above, the radiopaque marker is normally disposed only in the end portion of the stent. Consequently, the above-described procedures encounter difficulties. In addition, due to restenosis, a patient is transported to a hospital which is different from a hospital where the stent is caused to indwell, it is difficult to accurately recognize the overall shape of the stent. Consequently, there is a possibility that accurate treatment cannot be performed. In contrast, the stent according to the present invention can be introduced into the blood vessel while the overall shape is recognized under X-ray fluoroscopy. Therefore, it is possible to suppress or prevent risks during restenosis treatment by improving difficulties in the overlapping method or the two-stent method as described above. Therefore, the stent according to the present invention can be caused to safely and easily indwell the living body lumen. In addition, even if a small amount of the second metal material is provided, it is possible to achieve sufficient visibility. Accordingly, the thickness and the width of the stent can be reduced, and thus the stent is very useful in view of miniaturization of the stent which has been recently desired. Conventionally, there also exists a stent formed of an alloy including two or more metals. However, since the alloy normally maintains a uniform state, the conventional stent is different from the stent according to the present invention in terms of "dispersion" described in the present invention.

In the present invention, the potential of a metal (including a complete solid solution alloy) is referred to as a "corrosion potential". In this description, the "corrosion potential" is measured by using a method described in JIS T0302:2000, and specifically represents a value (mV) measured by the following method.

### <Method of Measuring Corrosion Potential>

A sample (sample configured to include metal) is cut into a suitable size. In order to eliminate an influence at the time of cutting, a sample is polished in running water using a waterproof polishing paper. In this case, the polishing paper is used in a range from the coarse one (150^{th}) to finally the 600^{th} one. After being polished, the sample is subjected to ultrasound cleaning in distilled water for five minutes. After being cleaned, a wire is connected to the sample. Depending on a shape of the sample, a method of connecting the sample and the wire to each other is appropriately selected from soldering, Dotite, clipping, screwing, and the like. Next, the sample including a connection portion with the wire is coated with a sealing agent so that the sample is exposed by the amount of only one square centimeter. After being coated therewith, the following evaluation is performed within 24 hours.

An electrolysis cell into which a PBS solution (composition: 8.0g/L NaCl, 0.2g/L KCl, 1.15g/L Na₂HPO₄, 0.2g/L KH₂PO₄, pH 7.2 to 7.6) is poured is placed into a constant temperature bath, and the temperature of the PBS solution is maintained at 37°C. High-purity nitrogen gas is subjected to bubbling in the PBS solution for 30 minutes or longer. Thereafter, the sample prepared as described above is immersed in the PBS solution. The potential generated in the sample is measured by using an electrometer (made by Hokuto Denko Co. , Ltd., Model No.: HE-104). The measurement is performed in a state where the sample is immersed at 37°C for one hour, and a value obtained after one hour is set to the corrosion potential (mV).

Hereinafter, an embodiment of the present invention will be described.

### [Stent]

The stent according to the present invention includes the sintered body of the base material that is formed of the first metal material with the second metal material that is dispersed in the base material. The stent according to the present invention may be either a balloon-expandable stent or a self-expandable stent.

A shape of the stent is not particularly limited as long as the stent has the above-described configuration. However, it is preferable that the stent has sufficient strength so as to stably indwell a living body lumen such as blood vessels or the like. For example, the shape preferably includes those which are formed with braided fibers in a cylindrical shape, or those which have an opening portion disposed in a tubular body.

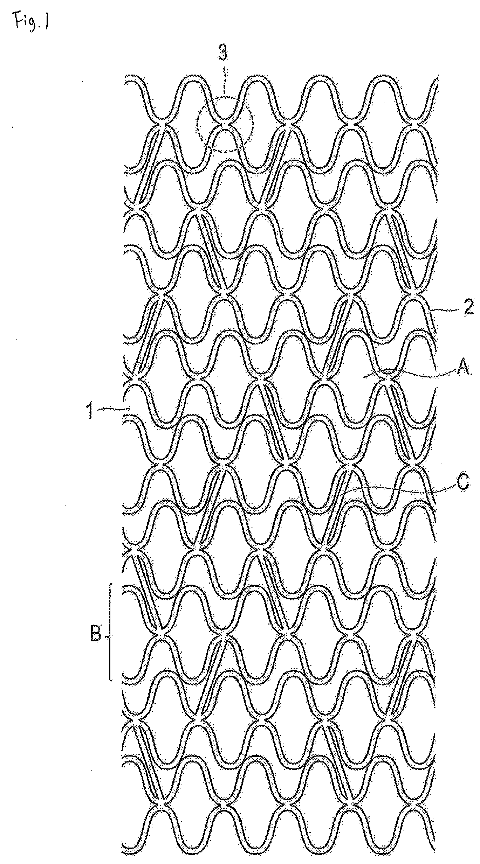

Hereinafter, as one embodiment of the stent according to the present invention, the stent illustrated in Fig. 1 will be described by exemplifying the balloon-expandable stent. As illustrated in Fig. 1, in a stent 1, a stent body (base material) 2 is a cylindrical body in which both end portions are open, and which extends between both the end portions in a longitudinal direction. A side surface of the cylindrical body has multiple cut-out portions which communicate between the outer surface and the inner surface. The stent has a structure which can be expanded and contracted in a radial direction of the cylindrical body by the cut-out portions being transformed. The stent is caused to indwell the living body lumen such as blood vessels or the like, and maintains the shape. In a form illustrated in Fig. 1, a basic unit of the stent body (base material) 2 includes substantially rhomboid-shaped elements A which internally have the cut-out portion. The multiple substantially rhomboid-shaped elements A are connected together while the substantially rhomboid shapes are arranged continuously in a minor axis direction thereof, thereby forming an annular unit B. The annular unit B is connected to the adjacent annular unit via a linear connection member C (this is also included in the concept of the "base material" in this description). In this manner, the multiple annular units B are arranged continuously in an axial direction thereof in a state where the multiple annular units B are partially connected together. According to this configuration, the stent body (base material) 2 forms the cylindrical body in which both end portions are open, and which extends between both the end portions in the longitudinal direction. Then, the side surface of the cylindrical body has a substantially rhomboid-shaped cut-out portion. The stent has the structure which can be expanded and contracted in the radial direction of the cylindrical body by the cut-out portion being transformed.

In the stent according to the present embodiment, the stent body (base material) 2 (including the connection member C) is formed of the first metal material, and is a sintered body which is sintered in a form in which the second metal material is dispersed in the stent body (base material) 2 (in Fig. 1, the second metal material is not illustrated). Although this dispersed form is not particularly limited, the second metal material may be dispersed so as to be scattered on a surface of the stent body (base material) 2 (connection member C), or may be dispersed in a sea-island state in an overall body (surface and inner portion) of the stent body (base material) 2. As described above, even if the second metal material is dispersed in the first metal material, a difference in the corrosion potential present between the first and second metal materials is small. Accordingly, a local current caused by the potential difference substantially hardly flows therein. Therefore, it is possible to suppress or prevent metal elution caused by galvanic corrosion.

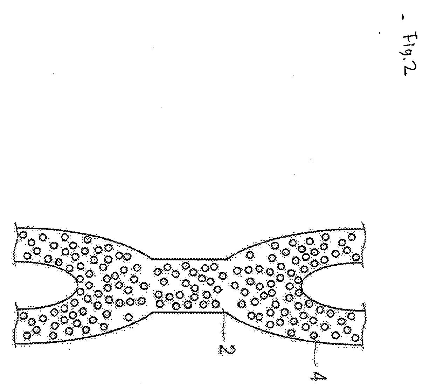

Fig. 2 is a partial enlarged view of a portion 3 surrounded by a dotted line in the stent illustrated in Fig. 1. As illustrated in Fig. 2, a second metal material 4 is dispersed in a sea-island state in the overall body (surface and inner portion) of the stent body (base material) 2 in the stent illustrated in Fig. 1. That is, preferably, the second metal material is dispersed in a sea-island state in the base material (at least a portion of the surface and/or the inner portion of the base material). Preferably, the second metal material is substantially uniformly dispersed in a sea-island state in the overall body (surface and inner portion) of the base material. As described above, in view of the fact that a position of the stent can be satisfactorily confirmed under X-ray fluoroscopy and the stent is miniaturized (film thickness and width are reduced), it is preferable to disperse the second metal in a sea-island state so as to be present not only on the surface of the stent body (base material) 2 but also in the inner portion. That is, as described above, in a case of the normal stent, the radiopaque metal material is separately installed in the end portion as a marker. In contrast, according to the present embodiment, in addition to an operational effect of suppressing/preventing the galvanic corrosion, the second metal material can be provided with an operational effect of functioning as the marker for more satisfactorily confirming the position of the overall stent. Therefore, there is an advantageous effect in that visibility of the stent becomes excellent without disposing a separate marker. At the same time, the overall shape of the stent can be simply confirmed, and the stent can be caused to more safely indwell the living body lumen. In addition, a material having a specific gravity higher than that of the first metal material (stent body (base material) 2) is selected as the second metal material 4, and the second metal material 4 is dispersed in a sea-island state so as to be present not only on the surface of the stent body (base material) 2 (including the connection member C) but also in the inner portion. In this manner, it is possible to increase the specific gravity per unit volume of the stent. Accordingly, compared to a form in which the second metal material 4 is scattered on only the above-described surface, an excellent effect is obtained in terms of visibility under X-ray fluoroscopy. According to the present invention, similarly to the conventional stent, a separate marker may be further disposed in the stent body (base material). This can further improve the visibility of the stent.

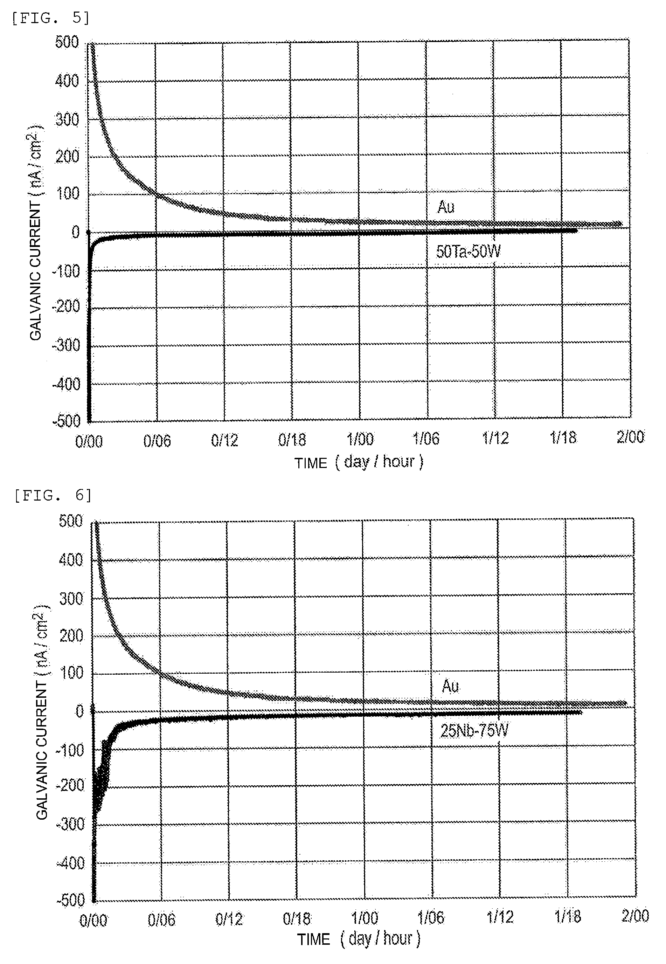

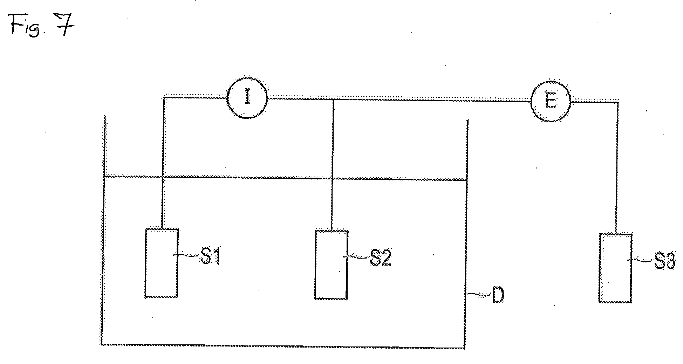

A structure of the stent according to the present invention is not limited to the form illustrated in Fig. 1, and a coil shape is also included in the concept of the present invention. A cross-sectional shape of the wire (that is, the base material) configuring the stent (main body) includes a rectangular shape, a circular shape, an elliptical shape, other polygonal shapes, or the like. However, other shape in addition to these shapes may be employed. In addition to the above-described configuration, as another preferred example of the stent 1, a stent having a lattice-like structure may be employed. In addition to these forms, for example, the present invention is similarly applicable to or can be appropriately modified for stents disclosed in JP-A-2002-263195 (corresponding to U.S. Patent Application Publication No. 2001/0001317) (particularly, refer to Figs. 9 and 10), JP-A-2004-121342 (corresponding to U.S. Patent Application Publication No. 2004/0127972) (particularly, refer to Fig. 6), JP-A-2004-290279 (particularly, refer to Figs. 6 and 7), JP-A-2005-278993, U.S. Patent No. 6,402,777 (particularly, refer to Fig. 1), or the like.

In addition, a size of the stent body (base material) is not particularly limited, and may be appropriately selected depending on applicable locations. Preferably, an outer diameter of the stent before being expanded is approximately 0.3 mm to 5 mm, more preferably, approximately 0.4 mm to 4.5 mm, and most preferably, approximately 0.5 mm to 1.6 mm. In addition, a length of the stent is also not particularly limited, and can be appropriately selected depending on diseases to be treated. For example, preferably, the length of the stent is approximately 5 mm to 100 mm, and more preferably, 6 mm to 50 mm. Alternatively, in some cases, preferably, the length of the stent is approximately 1.5 mm to 4 mm, and more preferably, 2 mm to 3 mm. In addition, a thickness of the stent is not particularly limited as long as the stent has the radial force needed to indwell the stenosis and does not hinder blood flow to some extent. However, for example, it is preferable that the thickness falls within a range of 1 µm to 1000 µm, and it is more preferable that the thickness falls within a range of 50 µm to 300 µm.

As described above, the second metal material is present in a dispersed state in the first metal material (stent body) . However, in this case, the shape of the second metal material is not particularly limited. Specifically, the shape of the second metal material includes a granular shape, a powder shape, a substantially spherical shape, a fibrous shape, a rod shape, a flat shape, an irregular shape, or the like. Among these shapes, it is preferable to employ the granular shape, the powder shape, or the substantially spherical shape, if visibility under X-ray fluoroscopy, convenient manufacturing, or the like is considered. In addition, the size of the second metal material is also not particularly limited. However, if visibility under X-ray fluoroscopy, manufacturing convenience, or the like is considered, for example, when the second metal material has the granular shape or the substantially spherical shape, an average particle diameter of the second metal material is preferably 0.01 µm to 100 µm, and more preferably, 0.1 µm to 30 µm. In this description, the term of the "particle diameter" means the maximum distance within a distance between any two points on a particle contour. A value of the "average particle diameter" employs a value calculated as an average value of the particle diameter of the particles observed in several to several tens of visual fields by using observation means such as a scanning electron microscope (SEM), a transmission electron microscope (TEM), or the like.

In addition, an abundance ratio in the stent of the second metal material is also not particularly limited. However, from the viewpoint of efficiently achieving an advantageous effect of the present invention, from the viewpoint of expandability of the stent, or from the viewpoint of fatigue strength, it is preferable that the second metal material is dispersed in a sea-island state in the base material at a rate of 10 vol% to 90 vol% with respect to the total volume of the stent. It is more preferable that the second metal material is dispersed in a sea-island state in the base material at a rate of 20 vol% to 50 vol% with respect to the total volume of the stent.

Here, the shapes of the first metal material and the second metal material which are used as a raw material in producing the stent are also not particularly limited. However, from the viewpoint of manufacturing the stent, it is preferable that the raw material is handled as powder. Specifically, the shapes include a spherical shape, a powder shape, a fibrous shape, a rod shape, a substantially spherical shape, a flat shape, an irregular shape, or the like. Among these shapes, it is preferable to employ the spherical shape, the powder shape, or the substantially spherical shape. The shapes of the first metal material and the second metal material after being produced as the stent are also not particularly limited. The shapes can be changed by appropriately adjusting sintering conditions thereof or the like.

In addition, the sizes of the first metal material and the second metal material which are used as a raw material are also not particularly limited. However, from the viewpoint of producing the stent or from the viewpoint of expandability as the stent, the size of the first metal material is preferably 0.01 µm to 200 µm, and more preferably, 0.1 µm to 50 µm. In addition, it is preferable that the size of the second metal material is substantially the same as or slightly smaller than the size of the above-described first metal material. Specifically, from the viewpoint of producing the stent or from the viewpoint of expandability of the stent, the size of the second metal material is preferably 0.01 µm to 200 µm, and more preferably, 0.1 µm to 50 µm. In order to prepare the metal material having the particle diameter within this predetermined range, the metal material may be classified by using a sieve or the like.

In addition, a weight ratio between the first metal material and the second metal material which are used as a raw material is also not particularly limited, and it is preferable that the weight ratio is the above-described abundance ratio of the second metal material in the stent. Specifically, from the viewpoint of radiopacity, it is preferable that the weight ratio between the first metal material and the second metal material is approximately 85:15 to 5:95, more preferably, approximately 70:30 to 30:70, and most preferably, approximately 65:35 to 35:65.

Subsequently, a specific example of components configuring the stent of the present invention such as the first metal material, the second metal material, and the like will be described.

### [First Metal Material]

The first metal material serves as the base material of the stent. In Fig. 1, the stent body 2 and the connection member C are formed of the first metal material. Without being particularly limited, as the first metal material, a metal can be used which is the same as the metal used for the stent in a conventional medical field. Specifically, the metal includes stainless steel, a cobalt-based alloy, a nickel-titanium-based alloy, tantalum, a tantalum-based alloy, platinum, a platinum-based alloy, gold, a gold-based alloy, molybdenum, a molybdenum-based alloy, or the like. In addition, pleating may be performed using the above-described metal material after the shape of the stent is produced using a material other than the above-described materials. Furthermore, after the final shape of the stent is produced, it is preferable to perform annealing. Performing the annealing improves flexibility and elasticity of the overall stent, thereby allowing the stent to satisfactorily indwell bent blood vessels. In addition, compared to a case where the annealing is not performed, the force of restoring a shape prior to expansion after the stent is expanded decreases, and in particular, the force of returning to a linear shape which appears when the stent is expanded in a portion of the bent blood vessel decreases. Accordingly, a physical stimulus applied to an inner wall of the bent blood vessel decreases, and thus it is possible to reduce factors of restenosis. It is preferable to perform the annealing by cooling the surface of the stent so as not to be oxidized at a speed which does not cause quenching and an intermediate phase appearing, after the stent is heated to 900°C to 1200°C in an inert gas atmosphere (for example, argon gas) or in vacuum. In addition, it is preferable to perform passivation treatment before and after the final shape of the stent is produced. Performing the passivation treatment causes a dense and thin oxide film to be formed on the surface of the stent. Accordingly, it is possible to more effectively suppress or prevent corrosion (elution) of the stent (metal), particularly corrosion (elution) of the stent (metal) in a body fluid.

Here, as the metal (first metal material) configuring the stent body, among the above-described materials, it is preferable to use the stainless steel, the cobalt-based alloy, the nickel-titanium-based alloy, or a mixture of these two or more materials. Here, as the stainless steel, stainless steel which can be used in the conventional medical field can be similarly used. For example, the stainless steel includes SS304, SS316L, SS420J2, SS630, or the like. From the viewpoint of corrosion resistance and actual usage records in a living body, among these materials, it is preferable to use SS316L (composition: carbon 0.035 wt% or less, phosphorous 0.04 wt% or less, sulfur 0.03 wt% or less, manganese 2.00 wt% or less, silicon 0.75 wt% or less, chromium 16.00 wt% to 18.00 wt%, nickel 12. 00 wt% to 15. 00 wt%, molybdenum 2.00 wt% to 3.00 wt%, and the remainder is iron). In addition, as the cobalt-based alloy, a cobalt-based alloy which can be used in the conventional medical field can be similarly used. For example, the cobalt-based alloy includes a cobalt-chromium alloy such as L605 and the like, a cobalt-nickel-chromium alloy, a cobalt-nickel-chromium-molybdenum alloy such as MP35N and the like, a cobalt-chromium-molybdenum alloy, or the like. From the viewpoint of corrosion resistance, strength, and actual usage records in a living body, among these materials, it is preferable to use L605 (composition: chromium 19.00 wt% to 21.00 wt%, nickel 9.00 wt% to 11.00 wt%, tungsten 14.00 wt% to 16.00 wt%, iron 3.00 wt% maximum, manganese 1.00 wt% to 2.00 wt%, carbon 0.05 wt% to 0.15 wt%, silicon 0.40 wt% maximum, phosphorous 0.040 wt% maximum, sulfur 0.030 wt% maximum, and the remainder is cobalt), or alternatively it is preferable to use MP35N (composition: carbon 0.025 wt% maximum, phosphorous 0.015 wt% maximum, sulfur 0.010 wt% maximum, manganese 0.15 wt% maximum, silicon 0.15 wt% maximum, chromium 19.00 wt% to 21.00 wt%, nickel 33.00 wt% to 37.00 wt%, molybdenum 9.00 wt% to 10.50 wt%, titanium 1.00 wt% maximum, boron 0.01 wt% maximum, iron 1.00 wt% maximum, and the remainder is cobalt). In particular, since L605 has high strength and high ductility, the stent is likely to be thinner and can easily be over-expanded. Thus, it is very preferable to use L605. In addition, as the nickel-titanium-based alloy, a nickel-titanium-based alloy which can be used in the conventional medical field can be similarly used. For example, the nickel-titanium-based alloy includes a nickel-titanium alloy which contains nickel of approximately 50 wt% to 60 wt% and the remainder of which is titanium, a nickel-titanium-copper alloy in which copper is added to the nickel-titanium alloy, or the like. From the viewpoint of actual usage records in a living body, corrosion resistance, and super-elastic properties, among these materials, it is preferable to use the nickel-titanium alloy (nitinol) which contains the above-described nickel of approximately 50 wt% to 60 wt% and the remainder of which is titanium. The above-described first metal material is a preferable material which is excellently balanced in strength and ductility as the stent material, and which enables reliable treatment for ensuring patency of the blood vessel while preventing the expanded blood vessel from being restenosed or occluded. The above-described metal alone (only one type) may configure the stent body, or a mixture of two or more types may configure the stent body. However, it is preferable that the above-described metal alone configures the stent body.

The first metal material (powder including the first metal material) used in the present invention may be bought from among those which are commercially available, or may be obtained through self-production. Even in a case of the self-production, the conventionally known knowledge may be referred to or the self-production may be performed in combination therewith. For example, the self-production may employ an atomizing method in which a molten form of a metal or an alloy is caused to flow out from a small hole of a bottom in a melting pot so as to become a streamlet, in which very fast air, nitrogen, argon, water, or the like is blown to the streamlet, and in which the molten metal is scattered and rapidly solidified, a mechanical alloying method of using a planetary ball mill, or the like.

### [Second Metal Material]

The second metal material is dispersed in the base material formed of the above-described first metal material. Here, the second metal material is preferably a complete solid solution alloy. The complete solid solution alloy is a complete solid solution. Accordingly, even if an alloy composition is changed, phase separation, precipitation, or the like does not occur therein. Therefore, it is possible to easily adjust a difference between the potential (corrosion potential) of the complete solid solution alloy and the potential (corrosion potential) of the first metal material so as to fall within a specific range, by adjusting the alloy composition of the complete solid solution alloy.

In addition, the second metal material has a specific gravity higher than that of the first metal material (has radiopacity higher than that of the first metal material). Here, the term of the "radiopacity" means that the metal material is visible to some degree under X-ray fluoroscopy in a conventional medical jobsite. In this description, density is used as an index of the radiopacity. Normally, as the density further increases, the radiopacity becomes higher. From the viewpoint of visibility under X-ray fluoroscopy in the normal medical jobsite, it is preferable that the second metal material has the density of 10g/cm³ or more, and it is more preferable that the second metal material has the density of 12g/cm³ or more. The second metal material, when this density (specific gravity) is provided therefor, can exhibit sufficient radiopacity, and is sufficiently visible under X-ray fluoroscopy. Although the upper limit of the density of the second metal material is not particularly limited, the density is generally 23g/cm³ or less, and is preferably 19g/cm³ or less.

The second metal material has a corrosion potential expressed by a corrosion potential of the first metal material ± 200 mV. The description of "the second metal material has a corrosion potential expressed by a corrosion potential of the first metal material ± 200 mV" means that an absolute value of a difference between the corrosion potentials of the first metal material and the second metal material is 200 mV or smaller, that is, the corrosion potential of the second metal material falls within a range from [corrosion potential of the first metal material - 200 mV] to [corrosion potential of the first metal material + 200 mV]. If the corrosion potential falls within this range, even when the first metal material and the second metal material are brought into contact with each other in a body fluid of the blood vessel or the like, a potential difference between both of these is small. Accordingly, it is possible to sufficiently suppress or prevent corrosion (galvanic corrosion) of the stent. The corrosion potential of the second metal material may be ± 200 mV of the corrosion potential of the first metal material. However, it is preferable that the corrosion potential of the second metal material is as close as possible to the corrosion potential of the first metal material. Therefore, preferably, the corrosion potential of the second metal material is ± 150 mV of the corrosion potential of the first metal material, more preferably ± 130 mV, much more preferably ± 120 mV, and most preferably ± 110 mV.

As long as the second metal material as described above has the specific gravity higher than that of the first metal material and has the corrosion potential expressed by the corrosion potential of the first metal material ± 200 mV, the second metal material may be configured to include any metal. Although not particularly limited, it is preferable that the second metal material is the complete solid solution alloy. Specifically, it is preferable that the second metal material is the complete solid solution alloy configured to include at least two metals selected from a group consisting of molybdenum (Mo), tantalum (Ta), niobium (Nb), tungsten (W), vanadium (V), cobalt (Co), palladium (Pd), nickel (Ni), and rhodium (Rh). A combination of these metals is not particularly limited as long as the combination forms the complete solid solution. For example, the combination includes Mo-Ta, Nb-W, Ta-W, Mo-W, Nb-Ta, V-W, Co-Pd, Mo-Nb, Mo-V, Ni-Pd, Ni-Rh, Nb-V, or the like. Among these combinations, it is preferable to use Mo-Ta, Nb-W, Ta-W, Mo-W, Nb-Ta, V-W, Co-Pd, Mo-Nb, Mo-V, Ni-Pd, and Ni-Rh. As long as the second metal material has the specific gravity higher than that of the first metal material and has the corrosion potential expressed by the corrosion potential of the first metal material ± 200 mV, the second metal material may be configured to include a single metal material (only one type) of the above-described metal materials or may be configured to include a mixture of two or more metal materials. However, it is preferable that the second metal material is configured to include the single metal material.

In addition, when the second metal material is the complete solid solution alloy, the composition is not particularly limited as long as the second metal material has the specific gravity higher than that of the first metal material and has the corrosion potential expressed by the corrosion potential of the first metal material ± 200 mV. The composition can be appropriately selected depending on the metal (first metal material) configuring the stent body. For example, when the first metal material is L605 which is subjected to passivation treatment, the composition is preferably 75Mo-25Ta, 50Mo-50Ta, 50Nb-50W, 25Nb-75W, and 50Ta-50W (each value is represented by mole%, and total values are 100 mole%). Alternatively, the composition is preferably 75Mo-25Ta, 50Mo-50Ta, 50Nb-50W, and 50Ta-50W (each value is represented by mole%, and total values are 100 mole%). The composition is most preferably 50Mo-50Ta, 50Nb-50W, and 50Ta-50W (each value is represented by mole%, and total values are 100 mole%). In addition, when the first metal material is SS316L which is subjected to passivation treatment, the composition is preferably 75Mo-25Ta, 50Mo-50Ta, 50Nb-50W, 25Nb-75W, and 50Ta-50W (each value is represented by mole%, and total values are 100 mole%). Alternatively, the composition is preferably 75Mo-25Ta, 50Mo-50Ta, 50Nb-50W, and 50Ta-50W (each value is represented by mole%, and total values are 100 mole%). The composition is preferably 50Mo-50Ta, 50Nb-50W, and 50Ta-50W (each value is represented by mole%, and total values are 100 mole%). When the second metal material is the complete solid solution alloy, the second metal material is allowed to contain other inevitable impurities (inevitable impurity elements) derived from its raw material. The inevitable impurities are components which are considered as unnecessary other than essential components configuring an alloy, and are impurities which are inevitably mixed therewith due to raw materials or processing steps of alloy components. The content of the above-described inevitable impurities is not particularly limited as long as the content does not significantly affect a normal alloy. However, the total content is preferably 0.3 mole% or less.

### [Binder]

The stent formed of the sintered body according to the present invention may include a binder from the viewpoint of the fact that the first metal material and the second metal material are brought into close contact with each other so as to increase the strength thereof or so as to minimize a crevice thereof. A type of the binder used for the stent according to the present invention is not particularly limited as long as the binder can be efficiently bound and can disappear after being sintered. For example, the binder includes an acrylic polymer and terpineol. As the acrylic polymer, those which are obtained by synthesis or those which are commercially available may be used. Those which are commercially available include OLYCOX KC series (sintering acrylic binder, manufactured by Kyoeisha Chemical Co. , Ltd.) and the like. The above-described binder may be used alone or may be used in a mixture form of two or more binders.

In addition, although the content of the binder is also not particularly limited, the content is preferably approximately 1 wt% to 30 wt% of the total weight of the first metal material and the second metal material, more preferably approximately 5 wt% to 25 wt%, and most preferably approximately 5 wt% to 20 wt%. When the binder is used in the mixture form of two or more binders, the content of the above-described binder is set to be a total amount of the binders.

Subsequently, a preferred embodiment of a manufacturing method of the stent according to the present invention will be described. First, the first metal material and the second metal material, and the binder if necessary are prepared. The second metal material is a metal which has the specific gravity higher than that of the first metal material and which has the corrosion potential expressed by the corrosion potential of the first metal material ± 200 mV. The first metal material and the second metal material may be respectively single independent metals, or may be an alloy thereof. For example, as the first and second metal materials, powder of the alloy may be prepared. As the first metal material, powder of the alloy may be prepared, and as the second metal material, powder of the single metal may be prepared. Alternatively, as the first metal material, powder of the single metal may be prepared, and as the second metal material, powder of the alloy may be prepared. A weight ratio in this case may also be appropriately set with reference to the above-described weight ratio between the first metal material and the second metal material. The prepared materials are mixed together well so as to be uniform. As a mixing method, it is preferable to perform mixing by using a powder mixing machine or the like. The uniformly mixed mixture is placed into a molding die of the stent so as to perform warm pressing. In this case, as conditions for the warm pressing, pressure is approximately 10 MPa to 30 MPa, temperature is approximately 200°C to 300°C, and a time is approximately one minute to five minutes. Thereafter, sintering is performed at the temperature of 700°C to 1000°C, in an inert gas atmosphere of Ar gas or the like or in vacuum, for approximately one hour to two hours. Thereafter, in order to further increase the density (in order to further eliminate the crevice), hot isostatic rolling (hot isostatic pressing method) (HIP: hot isostatic press) is performed. In this case, as conditions for the hot isostatic press, pressure is approximately 50 MPa to 300 MPa, temperature is approximately 800°C to 1200°C, and a time is approximately one hour to two hours. According to this HIP, the density is preferably set to 95 vol% or greater of the theoretical density, more preferably 97 vol% or greater, and most preferably 98 vol% or greater. In this way, the sintered body (rod) according to the present invention is produced. The warm pressing, the sintering, and the HIP may be performed once or may be repeatedly performed, if necessary. The condition of the sintering described herein is not limited to the above-described range, and can be appropriately changed so as to produce the sintered body in view of a melting point or a diffusion rate of the metal material. When the sintering is performed at a relatively low temperature, the sintering may be correspondingly performed for a long period of time, or may be performed using high pressure. In addition, when the sintering is performed at a high temperature, the required amount of time may be shortened. This condition can be appropriately set by those skilled in the art. In addition, for example, if those which have a high melting point are selected as the second metal material and the temperature condition is set in the range from the melting temperature of the first metal material to the melting temperature of the second metal material, the sintering can be performed without melting the second metal material while a shape at the time of preparation can be substantially maintained without any change. Alternatively, if the temperature condition is set so that both the first metal material and the second metal material are not melted, the sintering is performed while shapes when the first metal material and the second metal material are prepared are substantially maintained without any change. In this manner, it is possible to produce the sintered body (rod) in which surfaces of the metal materials are bound together in a solid phase. The rod produced in this manner is formed into a pipe shape by cutting off a center portion thereof through cutting process. This rod is cut into a pattern of the stent through laser processing, and chemical polishing and electrolytic polishing are further performed thereon. In this manner, it is possible to produce the stent.

### Embodiments

An advantageous effect according to the present invention will be described with reference to the following reference examples and embodiments.

### Reference Example 1

Alloys (complete solid solution alloys) including 75Mo-25Ta, 50Mo-50Ta, 50Nb-50W, 25Nb-75W, and 50Ta-50W are respectively produced. In the above description, each composition is represented by mole%.

The corrosion potential is measured for these alloys and gold (Au), and the result is illustrated in the following table 1. In addition, the corrosion potential was also measured for SS316L which was the metal configuring the commercially-available stent (Nobori (registered trademark), manufactured by Terumo Kabushiki Kaisha), and L605 (Co-Cr alloy) which was the metal configuring the commercially-available stent (Kaname (registered trademark), manufactured by Terumo Kabushiki Kaisha). The measurement results respectively showed - 90.80 mV and - 110.75 mV. Density (g/cm³), corrosion potential (mV), and a difference (mV) in the corrosion potential between SS316L and L605 in the above-described alloys are collectively illustrated in Table 1 below.

**[Table 1]**

| | Density (g/cm³) | Corrosion Potential (mV, v.s.SCE, PBS, 37°C) | Difference in Corrosion Potential (mV) | |
|---|---|---|---|---|
| | | | SS316L | L605 |
| 75Mo-25Ta | 12.0 | -217.68 | 126.9 | 106.9 |
| 50Mo-50Ta | 13.7 | -195.30 | 104.5 | 84.6 |
| 50Nb-50W | 13.6 | -191.85 | 101.1 | 81.1 |
| 25Nb-75W | 16.3 | -227.29 | 136.5 | 116.5 |
| 50Ta-50W | 17.9 | -200.74 | 109.9 | 90.0 |
| Au | 19.3 | 122.57 | 213.4 | 233.3 |
| SS316L | 7.95 | -90.80 | - | - |
| L605 | 9.10 | -110.75 | - | - |

According to Table 1 illustrated above, it is presumed that all complete solid solution alloys including 75Mo-25Ta, 50Mo-50Ta, 50Nb-50W, 25Nb-75W, and 50Ta-50W can suppress galvanic corrosion (elution of the stent body), while an absolute value of the difference in the corrosion potential with respect to SS316L or L605 which is the metal generally configuring the stent is 200 mV or smaller. In contrast, since in a case of gold (Au), the absolute value of the difference in the corrosion potential with respect to SS316L or L605 exceeds 200 mV, it is presumed that gold cannot suppress the galvanic corrosion (elution of the stent body).

### Reference Example 2

Alloys (complete solid solution alloys) including 75Mo-25Ta, 50Mo-50Ta, 50Ta-50W, and 25Nb-75W are respectively produced. In the above description, each composition is represented by mole%.

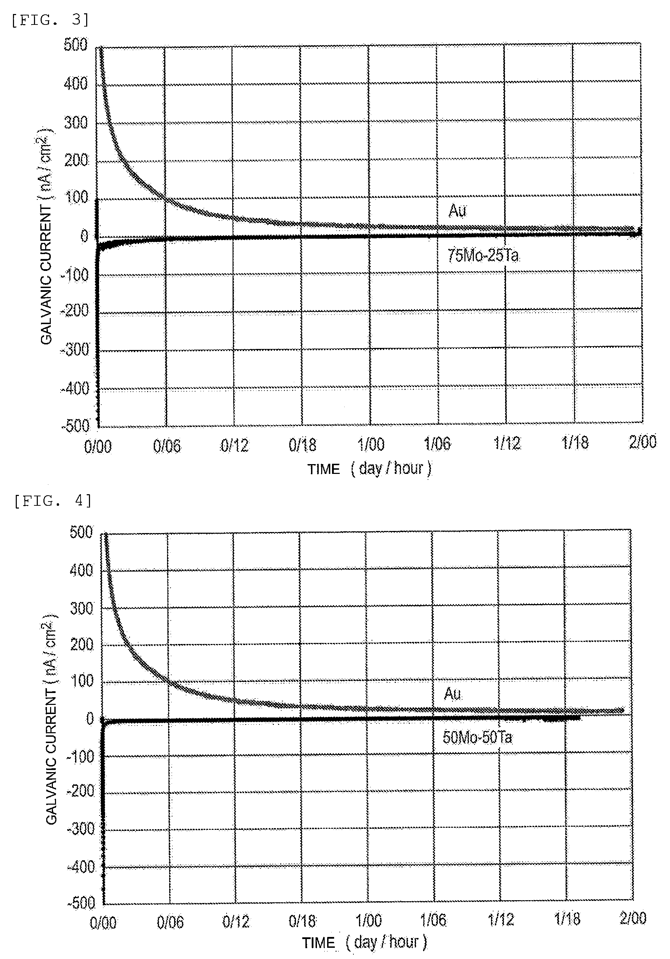

Each galvanic current of the above-described alloys and gold was measured by using the following method and a device illustrated in Fig. 7. The results are illustrated in Figs. 3 to 6. That is, a sample (sample including the complete solid solution alloy and gold) was cut into a suitable size (2 cm x 2 cm). After being cut, the sample was polished in running water using a waterproof polishing paper. In this case, the polishing paper was used in a range from the coarse one (150^{th}) to finally the 600^{th} one. After being polished, the sample was subjected to ultrasound cleaning in distilled water for five minutes. After being cleaned, a lead wire was connected to the sample by using a clip. Next, the sample including a connection portion with the lead wire or the like was coated with a sealing agent so that the sample was exposed by the amount of one square cm. This was used as a sample electrode (electrode S2). Similarly to the above-described manner, L605 (Co-Cr alloy) which was the metal configuring the commercially-available stent (Kaname (registered trademark), manufactured by Terumo Kabushiki Kaisha) was subjected to lead wire connection and sealing agent coating. This was used as a counter electrode (electrode S1). After the coating, the following evaluation was performed within 24 hours. At this time, as a reference electrode (standard electrode), an Ag/AgCl electrode (silver/silver chloride electrode) S3 was used, and an electrometer ("E" in Fig. 7) was installed between the reference electrode S3 and the electrodes S1 and S2. A cell D to which a PBS solution (composition: 8.0 g/L NaCl, 0.2g/L KCl, 1.15 g/L Na₂HPO₄, 0.2 g/L KH₂PO₄, pH 7.2 to pH 7.6) was poured was placed into a constant temperature bath (not illustrated), and the temperature of the PBS solution was maintained at 37°C. High-purity nitrogen gas was subjected to bubbling in the PBS solution for 30 minutes or longer. Thereafter, the electrodes S1 and S2 prepared as described above were immersed in the PBS solution. A current flowing between the counter electrode (electrode S1) and the sample electrode (electrode S2) was measured by using a zero shunt ammeter ("I" in Fig. 7). The temperature for measurement at this time was adjusted to 37°C.

As illustrated in Figs. 3 to 6, it was found that the alloy (complete solid solution alloys) including 75Mo-25Ta, 50Mo-50Ta, 50Ta-50W, and 25Nb-75W hardly generates or did not generate a galvanic current (local current) at all relative to L605 which was the metal generally configuring the stent. For this reason, it is considered that the stent having the first metal material and the second metal material according to the present invention can safely indwell a living body lumen for a long period of time without causing galvanic corrosion, even after being caused to indwell the living body lumen (for example, blood vessels). In contrast, it is presumed that gold which has a great potential difference with respect to the corrosion potential of the metal (L605) configuring the stent body generates the galvanic current (local current) relative to L605, and that the stent body is corroded (eluted). As illustrated in Figs. 3 to 6, in a case of gold, it is found that the galvanic current gradually decreases immediately after the gold is immersed. However, even in this case, corrosion (crevice corrosion) progressively occurs from a crevice between the stent body and the gold which is generated due to the galvanic corrosion. Accordingly, it is considered that the corrosion (elution) of the stent body is still in progress even after the galvanic current decreases.

### Example 1

Pure Mo powder and pure Ta powder are weighed, and 50Mo-50Ta alloy powder is produced using a planetary ball mill. Similarly, each powder such as pure Co, pure Cr, pure W, pure Ni, pure Mn, and the like is weighed, and L605 powder is produced by means of an atomizing method. The produced 50Mo-50Ta alloy powder and L605 powder are classified. Those which pass through a sieve of 10 µm within the 50Mo-50Ta alloy powder and those which pass through a sieve of 50 µm within the L605 powder are used.

Next, after the above-described classification, the 50Mo-50Ta alloy powder, the L605 powder, and the binder (weight ratio relative to metal powder 100 wt% shows 5wt% of OLYCOX KC-1100 and 20 wt% of terpineol) are mixed together (mixing weight ratio between the 50Mo-50Ta alloy powder and the L605 powder = 3:7), and are sintered at 900°C in the Ar atmosphere for one hour after the shape is produced through warm pressing at 30 MPa and 300°C for three minutes. Subsequently, hot isostatic press (HIP) is performed at 1200°C and 150 MPa for two hours so as to increase the density up to approximately 97 vol%.

The rod produced in this manner is formed into a pipe shape by cutting off a center portion thereof through cutting process. This rod is cut into a pattern of the stent through laser processing, and chemical polishing and electrolytic polishing are further performed thereon. In this manner, a stent is produced.

### Example 2

Pure W powder and pure Ta powder are weighed, and 50W-50Ta alloy powder is produced using a planetary ball mill. Similarly, each powder such as pure Co, pure Cr, pure W, pure Ni, pure Mn, and the like is weighed, and L605 powder is produced by using attritor. The produced 50W-50Ta alloy powder and L605 powder are classified. Those which pass through a sieve of 10 µm within the 50W-50Ta alloy powder and those which pass through a sieve of 50 µm within the L605 powder are used.

Next, after the above-described classification, the 50W-50Ta alloy powder, the L605 powder, and the binder (weight ratio relative to metal powder 100 wt% shows 5wt% of OLYCOX KC-1100 and 20 wt% of terpineol) are mixed together (mixing weight ratio between the 50W-50Ta alloy powder and the L605 powder = 4:6), and are sintered at 900°C in the Ar atmosphere for one hour after the shape is produced through warm pressing at 30 MPa and 300°C for three minutes. Subsequently, hot isostatic press (HIP) is performed at 1200°C and 150 MPa for two hours so as to increase the density up to approximately 97 vol%.

The rod produced in this manner is formed into a pipe shape by cutting off a center portion thereof through cutting operation. This rod is cut into a pattern of the stent through laser processing, and chemical polishing and electrolytic polishing are further performed thereon. In this manner, a stent (outer diameter: 1.0 mm, length: 18 mm, thickness: 60 µm) is produced. An abundance ratio of the second metal material (50W-50Ta alloy material) in the stent obtained in this way was a rate of 25 vol% with respect to the total volume of the stent.

The obtained stent is arranged inside a simulated blood vessel, and the stent is observed under X-ray fluoroscopy. As a result, the overall stent was satisfactorily visible.

Furthermore, the present application is based on Japanese Patent Application No. 2013-079325, filed on April 5, 2013, the disclosure of which is incorporated herein as a whole by reference.

## Claims

1. A stent comprising:
a sintered body of a base material that is formed of a first metal material with a second metal material that is dispersed in the base material,
wherein the second metal material has a specific gravity higher than that of the first metal material, and has a corrosion potential expressed by a corrosion potential of the first metal material ± 200 mV.

2. The stent according to Claim 1,
wherein the second metal material is a complete solid solution alloy.

3. The stent according to Claim 2,
wherein the complete solid solution alloy is configured to include at least two metals selected from a group consisting of molybdenum (Mo), tantalum (Ta), niobium (Nb), tungsten (W), vanadium (V), cobalt (Co), palladium (Pd), nickel (Ni), and rhodium (Rh).

4. The stent according to Claim 3,
wherein the complete solid solution alloy is selected from a group consisting of Mo-Ta, Nb-W, Ta-W, Mo-W, Nb-Ta, V-W, Co-Pd, Mo-Nb, Mo-V, Nb-Pd, and Ni-Rh.

5. The stent according to any one of Claims 1 to 4,
wherein the first metal material is selected from a group consisting of stainless steel, a cobalt-based alloy, and a nickel-titanium-based alloy.

6. The stent according to any one of Claims 1 to 5,
wherein the second metal material is dispersed in a sea-island state in the base material.

7. The stent according to Claim 6,
wherein the second metal material is dispersed in a sea-island state in the base material at a rate of 10 vol% to 90 vol% with respect to the total volume of the stent.
